# EUROPEAN PATENT APPLICATION

(11) **EP 4 763 363 A1**
(43) Date of publication of application: **24.06.2026**
(21) Application number: 25157373.9
(22) Date of filing: 12.02.2025
(51) Int. Cl.: B06B 1/02, B06B 1/06, A61M 11/00, A61M 15/00, B05B 17/00, B05B 17/06

(54) **METHOD OF SWEEPING FREQUENCY FOR MESH-TYPE NEBULIZER**

(30) Priority: 17.12.2024 CN 202411858685
(71) Applicant: Hcmed Innovations Co., Ltd., Taipei City 106 (TW)
(72) Inventor: CHEN, Yen-Ting, Taipei City (TW); TSAI, Chien-Shen, New Taipei City 235 (TW)
(74) Representative: Viering, Jentschura & Partner mbB Patent- und Rechtsanwälte

(57) **Abstract**

A method of sweeping frequency for a mesh-type nebulizer (1) includes the steps of: selecting an operating frequency range of a mesh unit (10); dividing the operating frequency range into a plurality of first detection frequency points with a first interval value; providing driving voltages sequentially to the plurality of first detection frequency points to determine a first optimal operating frequency; performing an algorithmic step on the first optimal operating frequency to obtain a fine sweep frequency section; dividing the fine sweep frequency section into a plurality of second detection frequency points by a second interval value; and providing driving voltages sequentially to the plurality of second detection frequency points to determine a selected optimal operating frequency. The first interval value is greater than or equal to the second interval value.

## Description

### FIELD OF THE INVENTION

The present invention relates to a method of sweeping frequency, and more particularly to a method for seeking and setting a resonant frequency for a mesh-type nebulizer.

### BACKGROUND OF THE INVENTION

Aerosol inhalation therapy has gradually become a popular way to treat human respiratory diseases. The types of nebulizers include mesh nebulizers, compressed air nebulizers, and ultrasonic nebulizers. Among them, the vibrating mesh nebulizer uses high-frequency vibration of a mesh to cause the medicinal liquid to pass through small holes on the mesh to form atomized particles, which are inhaled into human body through an inhalation device, such as a mask or a mouthpiece.

In order to ensure that the mesh is oscillated at the optimal operating frequency point for the drug delivery, existing mesh nebulizers usually oscillate the mesh at a fixed frequency value and then oscillate the mesh at increasing or decreasing frequencies within a specific operating frequency range prior to aerosol delivery treatment of the drug. Next, an optimal operating frequency point is determined after oscillation tests are completed at all operating frequency points within the operating frequency range. Finally, after the optimal operating frequency point has been determined, the mesh nebulizer will oscillate at the determined optimal operating frequency point for drug delivery.

However, the existing technology requires all operating frequency points within a specific operating frequency range to be oscillated, and then the optimal operating frequency point can be determined. Such results in a longer total time for scanning all operating frequency points, and the oscillation test for each operating frequency point is accompanied by the atomization of the liquid medicine, which requires the consumption of more liquid medicine and results in a waste of liquid medicine.

Therefore, how to improve a process of testing a proper operating frequency for a mesh-type nebulizer through a proper improvement, so as to overcome the above-mentioned deficiencies, has become one of the important issues to be solved in this industry.

### SUMMARY OF THE INVENTION

In response to the above-referenced technical inadequacies, the present invention provides a method of sweeping frequency for a mesh-type nebulizer to solve the above-mentioned problems.

In order to solve the above-mentioned problems, one of the technical aspects adopted by the present invention is to provide a method of sweeping frequency for a mesh-type nebulizer, which includes the steps of: selecting an operating frequency range of a mesh unit of the mesh-type nebulizer; dividing the operating frequency range into a plurality of first detection frequency points by a first interval value; providing driving voltages sequentially to the first detection frequency points in order to determine a first optimal operating frequency; performing an algorithmic step on the first optimal operating frequency to obtain a fine sweep frequency section; dividing the fine sweep frequency section into a plurality of second detection frequency points by a second interval value; and sequentially providing driving voltages to the plurality of second detection frequency points to determine a selected optimal operating frequency; wherein the first interval value is greater than or equal to the second interval value.

Therefore, in the method of sweeping frequency for the mesh-type nebulizer provided by the present invention, in which a first optimal operating frequency is determined within a fixed operating frequency range by a first interval value, and then, based on the first optimal operating frequency, to perform a test to determine a selected optimal operating frequency by a second interval (frequency) value, wherein the first interval (frequency) value is longer then the second interval (frequency) value. The advantage of this method is that the time required to confirm the operating frequency point (i.e., when the mesh unit reaches resonance) is reduced, and on the other hand, the amount of medicine consumed to confirm the operating frequency point is reduced.

These and other aspects of the present invention will become apparent from the following description of the embodiment taken in conjunction with the following drawings and their captions, although variations and modifications therein may be affected without departing from the spirit and scope of the novel concepts of the invention.

### BRIEF DESCRIPTION OF THE DRAWINGS

The described embodiments may be better understood by reference to the following description and the accompanying drawings, in which:
FIG. 1 is a schematic view of a mesh-type nebulizer according to the present invention;
FIG. 2 is a flowchart of a method of sweeping frequency for the mesh-type nebulizer according to the present invention;
FIG. 3 is a graph showing a first frequency sweeping by the method of sweeping frequency for the mesh-type nebulizer according to the present invention; and
FIG. 4 is a graph showing a second frequency sweeping by the method of sweeping frequency for the mesh-type nebulizer according to the present invention.

### DETAILED DESCRIPTION OF THE EXEMPLARY EMBODIMENTS

The present invention is more particularly described in the following examples that are intended as illustrative only since numerous modifications and variations therein will be apparent to those skilled in the art. Like numbers in the drawings indicate like components throughout the views. As used in the description herein and throughout the claims that follow, unless the context clearly dictates otherwise, the meaning of "a," "an" and "the" includes plural reference, and the meaning of "in" includes "in" and "on." Titles or subtitles can be used herein for the convenience of a reader, which shall have no influence on the scope of the present invention.

The terms used herein generally have their ordinary meanings in the art. In the case of conflict, the present document, including any definitions given herein, will prevail. The same thing can be expressed in more than one way. Alternative language and synonyms can be used for any term(s) discussed herein, and no special significance is to be placed upon whether a term is elaborated or discussed herein. A recital of one or more synonyms does not exclude the use of other synonyms. The use of examples anywhere in this specification including examples of any terms is illustrative only, and in no way limits the scope and meaning of the present invention or of any exemplified term. Likewise, the present invention is not limited to various embodiments given herein. Numbering terms such as "first," "second" or "third" can be used to describe various components, signals or the like, which are for distinguishing one component/signal from another one only, and are not intended to, nor should be construed to impose any substantive limitations on the components, signals or the like.

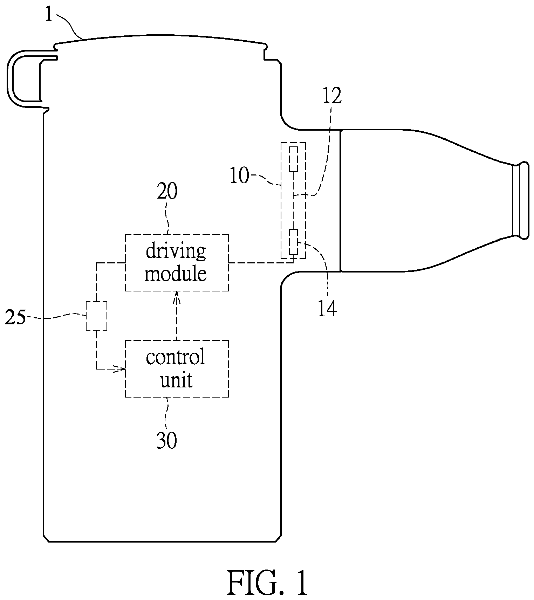

Referring to FIG. 1 to FIG. 4, an embodiment of the present invention provides a method of sweeping frequency for a mesh-type nebulizer, which is adapted to a mesh-type nebulizer 1 to seek a proper operating frequency. In general, the mesh-type nebulizer 1 includes a mesh unit 10, a driving module 20, a control unit 30 and a voltage feedback unit 25. The driving module 20 is connected between the mesh unit 10, the voltage feedback unit 25 and the control unit 30, so that the driving module 20, the control unit 30, the voltage feedback unit 25 and the mesh unit 10 are electrically connected to each other. In this embodiment, the control unit 30 can be a microcontroller unit (MCU), which can sequentially output an operating frequency to the driving module 20. In this embodiment, the driving module 20 is a power amplification unit to amplify the signal output by the control unit 30, and then drive the mesh unit 10. It should be noted that in other embodiments, the driving module 20 can also be directly installed on the control unit 30 to drive the mesh unit 10.

The method of sweeping frequency for the mesh-type nebulizer 1 includes the following steps.

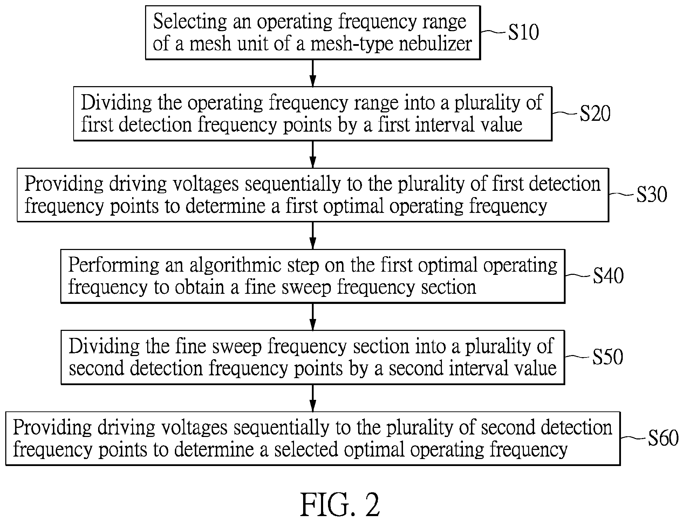

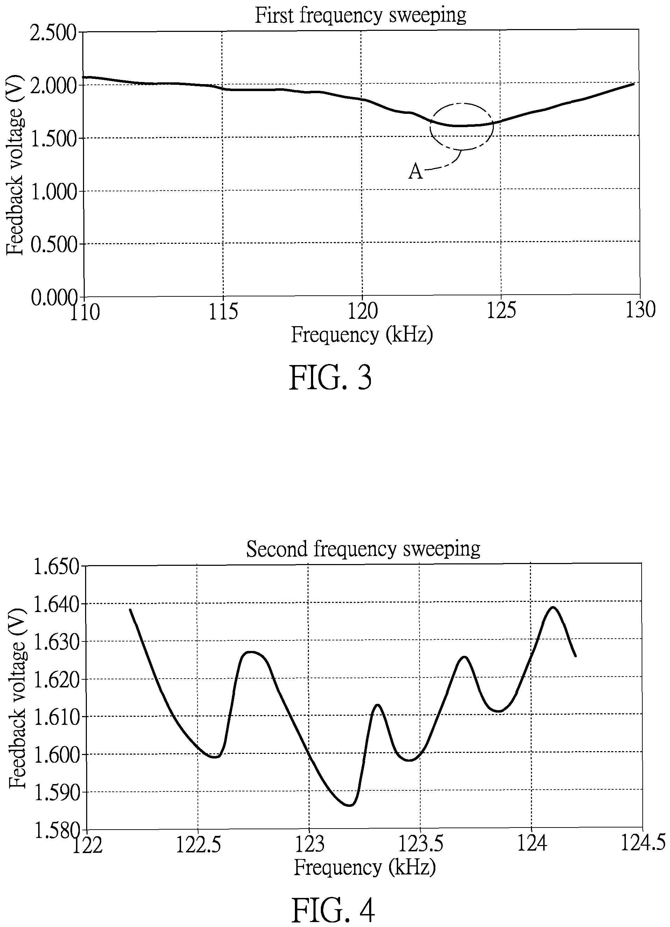

First, as shown in FIGS. 1 and 2, in step S10, an operating frequency range of the mesh unit 10 of the mesh-type nebulizer 1 is selected. The operating frequency range of the mesh unit 10 is usually determined by the piezoelectric ceramic material and specifications of the piezoelectric element. This operating frequency range is known during the design and manufacturing process of the mesh unit 10. Piezoelectric ceramic material is a functional ceramic material that can convert mechanical energy and electrical energy into each other. Specifically, the mesh unit 10 of the embodiment includes a mesh 12 and a hard ceramic piezoelectric element 14. The hard ceramic piezoelectric element 14 is made of hard piezoelectric ceramics. The mesh 12 is connected to the hard ceramic piezoelectric element 14 and driven by the hard ceramic piezoelectric element 14. For example, as shown in FIG. 3, the operating frequency range of the mesh unit 10 in this embodiment is between 110 kHz - 130 kHz, as the range on the horizontal axis. This operating frequency range can be regarded as a coarse sweep operating frequency range of rough scanning.

Due to the influence of different doping materials (lead zirconated titanate materials), piezoelectric ceramics are usually classified into two categories of soft and hard. Soft piezoelectric ceramics have higher electrical coupling coefficients, lower coercive fields and lower Mechanical Quality Factor (Qm). Soft piezoelectric ceramics can provide relatively large strain outputs, which are often suitable for applications such as flow meters, liquid level sensors, sensing and actuation.

The reason and advantage of using hard ceramic piezoelectric element 14 in this embodiment is that hard piezoelectric ceramics have lower electrical coupling coefficient, higher coercive field and higher Mechanical Quality Factor (Qm). Hard piezoelectric ceramics are commonly used in high-power applications. Specifically, the Mechanical Quality Factor (Qm) represents the performance of a piezoelectric material during mechanical vibration or resonance, and represents the efficiency of the piezoelectric material in terms of mechanical strain and mechanical energy conversion (i.e., energy loss). In high-power converter applications, a higher mechanical quality factor is required.

Referring to FIGS. 2 and 3, next, in step S20, the operating frequency range is divided into a plurality of first detection frequency points by a first interval value. In other words, the operating frequency range of 110 kHz - 130 kHz is divided into a plurality of first detection frequency points by a fixed interval value. In this embodiment, the first interval value can be between 0.1 kHz and 10 kHz. If the first interval value is too small, it will cause too many detection times, which will take longer time and waste more liquid medicine for testing. On the other hand, if the first interval value is too large, the detection will be rough and may miss the precision range.

For example, if the first interval value is 0.1 kHz, the total number of first detection frequency points in this embodiment is (130-110)/0.1=200, including the first starting value of 110 kHz. The control unit 30 of the mesh-type nebulizer 1 outputs a plurality of first detection frequency points in sequence, which are 110 kHz, 110.1 kHz, 110.2 kHz, 110.3 kHz, ... 129.8 kHz, 129.9 kHz, and 130 kHz.

In another practical way, if the first interval value is 1 kHz, the total number of first detection frequency points in this example is (130-110)/1=20, including the first starting value of 110 kHz. The control unit 30 of the mesh-type nebulizer 1 outputs a number of first detection frequency points in sequence, which are 110 kHz, 111 kHz, 112 kHz, 113 kHz, ...128 kHz, 129 kHz, and 130 kHz.

In step S30, provide driving voltages to the plurality of first detection frequency points in sequence to determine a first optimal operating frequency. In this embodiment, the control unit 30 is used to sequentially send the plurality of first detection frequency points to the driving module 20, which drives the mesh unit 10 according to the plurality of first detection frequency points, and then the voltage feedback unit 25 is used to receive a plurality of first feedback voltage values generated by the driving module 20. By comparing the values of the plurality of first feedback voltage, when a received one of the first feedback voltage values is the minimum value, a corresponding one of the first detection frequency points is set as the first optimal operating frequency.

Specifically, the control unit 30 sequentially outputs the above-mentioned first detection frequency points, which are amplified by the driving module 20, to detectively drive the hard ceramic piezoelectric element 14 of the mesh unit 10 to act. The voltage feedback unit 25 utilized in this example, for example, can be a voltage signal processor, which is fed back to the control unit 30 by the driving module 20 through the voltage feedback unit 25. According to an electrical relationship of the hard ceramic piezoelectric element 14 when operating in the resonance state, that the impedance (Z) is the smallest, the current (I) is the largest, and the power (P) is the largest. Therefore, the voltage feedback unit 25 can detect the change of the driving signal (voltage V) of the hard ceramic piezoelectric element 14 during the frequency sweeping process so as to generate an analog voltage feedback signal. The smaller the value of the voltage feedback signal, the closer the operating frequency of the mesh unit 10 is or equal to the resonant frequency. The control unit 30 sequentially sends a plurality of first detection frequency points to the driving module 20, and the voltage feedback unit 25 receives a plurality of first feedback voltage values generated by the driving module 20. By comparing the values of the plurality of first feedback voltages, one of the first detection frequency points can be selected as the overall optimal operating frequency of the mesh unit 10.

For example, as shown in FIG. 3, after the first frequency sweep (coarse sweep), the operating frequency range is set between 110 kHz - 130 kHz, and the first interval value is set to 1 kHz. When the first detection frequency point is around 123 kHz, the first feedback voltage value is the minimum value, which is approximately 1.6V. Therefore, this embodiment can take the first detection frequency point 123 kHz as the first optimal operating frequency.

It should be noted that the above-mentioned detected feedback signal can also be a current feedback signal. According to the electrical relationship of the mesh unit 10 when operating in the resonance state, the impedance (Z) is the smallest, the current (I) is the largest, and the power (P) is the largest. The change of the driving signal (current I) of the mesh unit 10 during the frequency sweep process can be detected to generate an analog current feedback signal. The larger the signal value of the current feedback signal, the closer the overall operating frequency of the mesh unit 10 is, or equal to the resonant frequency.

In step S40, an algorithmic step is performed on the first optimal operating frequency to obtain a fine sweep frequency section. The fine sweep frequency section is the first optimal operating frequency plus or minus a test interval value, and the test interval value is between 1 kHz and 3 kHz (the plus or minus value may be different). For example, the first optimal operating frequency of this embodiment is 123 kHz, and the test interval value can be equal to the above first interval value (e.g., 1 kHz), or slightly greater than the first interval value (e.g., 1.5 kHz). If the test interval value is 1 kHz, the fine sweep frequency section is 123 kHz ± 1 kHz. In other words, the fine sweep frequency section is 122 kHz to 124 kHz. If the test interval value is 1.5 kHz, the fine sweep frequency section is 123 kHz ± 1.5 kHz. In other words, the fine sweep frequency section is 121.5 kHz to 124.5 kHz. However, the positive and negative values of the test interval can be different, e.g., minus 1 kHz, and plus 1.5 kHz. In this embodiment, the range of the fine sweep frequency section is set from 122 kHz to 124.5 kHz as shown in the horizontal axis of FIG. 4.

In step S50, the fine sweep frequency section is divided into a plurality of second detection frequency points by a second interval value. The first interval value (frequency interval) is greater than or equal to the second interval value (frequency interval). In other words, the fine sweep frequency section 122 kHz to 124.5 kHz is further divided into multiple detection frequency points by a fixed interval value. In this embodiment, the second interval value is between 0.01 kHz and 0.5 kHz. Similar to the above-described, if the second interval value is too small, it will result in too many times of detection, longer time consuming, and more waste of the test liquid medicine. On the other hand, if the second interval value is too large, it will result in a rough detection, and may miss the precision range.

For example, if the second interval value is 0.2 kHz, the total number of second detection frequency points in this example is (124.5-122)/0.2, which is about 12, and the first starting value can be taken as 122 kHz. The control unit 30 of the mesh-type nebulizer 1 outputs a number of second detection frequency points in sequence, which are 122 kHz, 122.2 kHz, 122.4 kHz, 124.0 kHz, 124.2 kHz, 124.2 kHz, 124.0 kHz, 124.2 kHz, ...... 124.0 kHz, and 124.2 kHz.

On the other hand, if the second interval value is 0.4 kHz, the total of the second detection frequency points in this embodiment is (124.5-122)/0.4, which can be an integer of 5 to 7. For example, the first starting value can be 122 kHz. The control unit 30 of the mesh-type nebulizer 1 sequentially outputs a plurality of second detection frequency points, which are 122 kHz, 122.4 kHz, 122.8 kHz, 123.2 kHz, 123.6 kHz, 124.0 kHz, and 124.4 kHz.

It should be noted that the second interval value is preferably a common factor of twice the test interval value (for example, plus or minus 1 kHz). In this embodiment, it is a common factor of 2 kHz, so that an integer number of second detection frequency points can be obtained.

Finally, in step S60, driving voltages are sequentially provided to the plurality of second detection frequency points to determine a selected optimal operating frequency. Similar to step S30, the control unit 30 is used to sequentially send out a plurality of second detection frequency points. The driving module 20 drives the mesh unit 10 according to the plurality of second detection frequency points. Then, the voltage feedback unit 25 receives a plurality of second feedback voltage values generated by the driving module 20. By comparing the plurality of second feedback voltages, when the received second feedback voltage value is the smallest value, the corresponding second detection frequency is selected as the optimal operating frequency.

For example, as shown in Fig. 4, after the second sweep (fine sweep), the frequency range of the fine sweep is between 122 kHz and 124.5 kHz. After the fine sweeping for the whole frequency range, the second feedback voltage is minimized at 123.2 kHz, which is about 1.586 V. Therefore, the second detection frequency point of 123.2 kHz can be selected as the optimal operating frequency for this example.

In this embodiment, the method of sweeping frequency for the mesh-type nebulizer can be re-executed every time when the mesh-type nebulizer is turned on, so as to avoid mechanical aging affecting the resonance frequency of the hard ceramic piezoelectric element 14. At the same time, it can ensure that the hard ceramic piezoelectric element 14 of the mesh unit 10 is operating in a resonant state. Therefore, the stability of the atomization efficiency can be improved.

The method of sweeping frequency of the mesh-type nebulizer of the present invention is based on two times of frequency sweeping: a coarse sweep and a fine sweep. The examples are described above. However, the number of frequency sweeps of the present invention is not limited to two times.

In detail, in some cases, during the coarse sweeping process, the hard ceramic piezoelectric element 14 of the mesh unit 10 may have two resonant frequencies. Probably, due to the sampling of the first interval value, it may miss a lower first optimal operating frequency.

Thus, for example, the process of performing the algorithmic step on the first optimal operating frequency may also include obtaining an alternative operating frequency range. The alternative operating frequency range is divided into a plurality of third detection frequency points. And, a plurality of driving voltages is sequentially provided to the plurality of third detection frequency points to determine another alternative optimal operating frequency. Specifically, the process of performing this algorithmic step, that is, in the step S40, the second lowest first feedback voltage value can also be used. For example, the curve in FIG. 3 may produce two valleys, and the frequency value of the other valley can be used as another optimal operating frequency to obtain the alternative operating frequency range.

### [Beneficial Effects of the Embodiments]

In conclusion, in the method of sweeping frequency for the mesh-type nebulizer provided by the present invention, in which a first optimal operating frequency is determined within a fixed operating frequency range with a first interval value, and then, based on the first optimal operating frequency, to perform a test to determine a selected optimal operating frequency with a second interval (frequency) value, wherein the first interval (frequency) value is longer then the second interval (frequency) value. The advantage of this method is that it reduces the time required to confirm the operating frequency point (i.e., when the mesh unit reaches resonance), and on the other hand, it reduces the amount of drug consumed to confirm the operating frequency point.

On the other hand, the method of sweeping frequency for the mesh-type nebulizer of the present invention can be re-executed every time when the mesh-type nebulizer is turned on, so as to improve the stability of the nebulizing efficiency of the mesh unit. In addition, the mesh-type nebulizer of the present invention emits a more stable liquid, which ensures that each hole of the mesh (or orifice) is filled with liquid.

Compared with the previous technology, the previous method sweeps one time in a fixed range, the sweep interval is shorter and the number of testing is more. A longer time is spent on confirming the operating frequency point of the mesh unit. On the other hand, confirming the operating frequency point of the mesh unit also consumes more medicine.

The foregoing description of the exemplary embodiments of the invention has been presented only for the purposes of illustration and description and is not intended to be exhaustive or to limit the invention to the precise forms disclosed. Many modifications and variations are possible in light of the above teaching.

The embodiments were chosen and described in order to explain the principles of the invention and their practical application so as to enable others skilled in the art to utilize the invention and various embodiments and with various modifications as are suited to the particular use contemplated. Alternative embodiments will become apparent to those skilled in the art to which the present invention pertains without departing from its spirit and scope.

## Claims

1. A method of sweeping frequency for a mesh-type nebulizer (1), **characterized by** comprising the steps of:
selecting an operating frequency range of a mesh unit (10) of the mesh-type nebulizer (1);
dividing the operating frequency range into a plurality of first detection frequency points by a first interval value;
providing driving voltages sequentially to the first detection frequency points in order to determine a first optimal operating frequency;
performing an algorithmic step on the first optimal operating frequency to obtain a fine sweep frequency section;
dividing the fine sweep frequency section into a plurality of second detection frequency points by a second interval value; and
sequentially providing driving voltages to the plurality of second detection frequency points to determine a selected optimal operating frequency;
wherein the first interval value is greater than or equal to the second interval value.

2. The method according to claim 1, wherein the mesh unit (10) comprises a hard ceramic piezoelectric element (14), and a mesh (12), the hard ceramic piezoelectric element (14) is made of hard piezoelectric ceramic, and the mesh (12) is connected to the hard ceramic piezoelectric element (14) and is driven by the hard ceramic piezoelectric element (14).

3. The method according to claim 1, wherein the first interval value is between 0.1 kHz and 10 kHz.

4. The method according to claim 3, wherein the second interval value is between 0.01 kHz and 0.5 kHz.

5. The method according to claim 1, wherein the fine sweep frequency section is the first optimal operating frequency plus or minus a test interval value.

6. The method according to claim 5, wherein the test interval value is between 1 kHz and 3 kHz.

7. The method according to claim 1, wherein the mesh-type nebulizer (1) has a driving module (20), a control unit (30) and a voltage feedback unit (25), the driving module (20), the control unit (30), the voltage feedback unit (25) and the mesh unit (10) are electrically connected to each other, the control unit (30) is configured to sequentially provide the plurality of first detection frequency points to the driving module (20), the driving module (20) drives the mesh unit (10) according to the plurality of first detection frequency points, and the voltage feedback unit (25) is configured to receive a plurality of first feedback voltage values generated by the driving module (20); wherein, by comparing the plurality of the first feedback voltage values, when one of the first feedback voltage values is the smallest, the corresponding one of the first detection frequency points is considered as the first optimal operating frequency.

8. The method according to claim 7, wherein the control unit (30) is configured to sequentially send out the plurality of second detection frequency points, the driving module (20) is configured to drive the mesh unit (10) according to the plurality of second detection frequency points, the voltage feedback unit (25) is configured to receive a plurality of second feedback voltage values generated by the driving module (20), and
wherein, by comparing the plurality of second feedback voltage values, when the second feedback voltage value is the smallest, the corresponding one of the second detection frequency points is selected as the selected optimal operating frequency.

9. The method according to claim 7, wherein the process of performing the algorithmic step on the first optimal operating frequency further comprises the steps of:
obtaining an alternative operating frequency range;
dividing the alternative operating frequency range into a plurality of third detection frequency points by the second interval value; and
providing driving voltages sequentially to the plurality of third detection frequency points to determine an alternative optimal operating frequency.

10. The method according to claim 9, wherein, when performing the process of the algorithmic step, the alternative operating frequency range is obtained based on a second lowest one of the first feedback voltage values.

11. The method according to claim 1, wherein the method is re-executed every time, when the mesh-type nebulizer (1) is turned on.
